# EUROPEAN PATENT APPLICATION

(11) **EP 4 269 620 A1**
(43) Date of publication of application: **01.11.2023**
(21) Application number: 22169795.6
(22) Date of filing: 25.04.2022
(51) Int. Cl.: C12Q 1/6883

(54) **METHODS, DEVICES AND SYSTEMS FOR DETERMINING A PRESENCE OR ABSENCE OF GENETIC MARKERS OF RHEUMATOID ARTHRITIS AND DETERMINING A RISK OF DEVELOPING RHEUMATOID ARTHRITIS IN AN INDIVIDUAL**

(71) Applicant: Phadia GmbH, 79111 Freiburg (DE)
(72) Inventor: GEHRING, Isabel, 77652 Offenburg (DE); GRÜNDHUBER, Maria Theresa, 81547 München (DE)
(74) Representative: Brann AB

(57) **Abstract**

The present invention relates to a method for indicating the presence or absence of rheumatoid arthritis in an individual comprising determining the presence of one or more of specific pairs of genetic variants in a biological sample obtained from said individual. The invention also relates to a computer-implemented method of determining a level of risk for developing rheumatoid arthritis in an individual comprising correlating the presence of at least one of the pairs of genetic variants with an increased risk of the individual developing rheumatoid arthritis as compared with a risk of the individual developing rheumatoid arthritis in the absence of all of the pairs of genetic variants in the biological sample. Each of the specific pairs of genetic variants have been discovered to be indicative of an elevated risk for the individual to develop RA.

## Description

### FIELD OF THE INVENTION

The present invention is directed to methods for indicating the presence or absence of rheumatoid arthritis in an individual by determining the presence or absence of one or more pairs of genetic variants, also referred to as genetic markers, related to rheumatoid arthritis in said individual. The present invention also relates to computer-implemented methods of determining a level of risk for developing rheumatoid arthritis in an individual based on the presence or absence of said genetic markers. The invention is also directed to assay panels for detecting the genetic markers and to assay systems for use in the methods disclosed herein.

### BACKGROUND OF THE INVENTION

Rheumatoid arthritis (often abbreviated RA) is a systemic autoimmune disease that can lead to the destruction of joints and loss of mobility (see www.rheumatology.org/I-Am-A/Patient-Caregiver/Diseases-Conditions/Rheumatoid-Arthritis). Intra-articular bone erosion usually occurs early during disease progression with about 30 percent of rheumatoid arthritis patients having radiographically confirmed bone erosions at diagnosis and about 70 percent of rheumatoid arthritis patients having radiographically confirmed bone erosions three years after diagnosis. The early differential diagnosis of rheumatoid arthritis from clinically similar diseases, for example, osteoarthritis, is needed because the treatment and outcomes differ greatly between these two common forms of arthritis. Early initiation of disease-modifying antirheumatic drug (DMARD) treatment in rheumatoid arthritis is more effective in modulating the erosive and persisting nature of rheumatoid arthritis as compared with delayed initiation of DMARD treatment (van der Heide et al., The effectiveness of early treatment with "second-line" antirheumatic drugs. A randomized, controlled trial. Annals of Internal Medicine, 1996; 124). However, the median time for a patient to start DMARD treatment from onset of symptoms has been estimated at almost 12 months and typically after significant joint damage has occurred. Accordingly, early diagnosis of rheumatoid arthritis is critical for the initiation of treatment that can slow down the disease progression, prevent bone erosion, and improve a patient's quality of life.

Currently, the diagnosis of RA is typically aided or assisted by the presence of higher than normal levels of the serologic biomarkers anti-cyclic citrullinated peptide immunoglobulin G (CCP IgG) and/or rheumatoid factor immunoglobulin M (RF IgM). The sensitivity using CCP IgG as a biomarker for rheumatoid arthritis is about 68% (using CCP IgG correctly returns a positive result for about 68% of people who have the disease, but returns a negative result, i.e., a false-negative, for about 32% of the people who have the disease and should have tested positive) while the specificity using CCP IgG as a biomarker for rheumatoid arthritis is higher than 96% (using CCP IgG correctly returns a negative result for higher than 96% of people who do not have the disease, but returns a positive result, i.e., a false-positive, for almost 4% of the people who do not have the disease and should have tested negative). Using RF IgM as a biomarker for rheumatoid arthritis, the sensitivity is about 75% with a specificity of about 74% (Bas et al., Diagnostic tests for rheumatoid arthritis: comparison of anti-cyclic citrullinated peptide antibodies, anti-keratin antibodies and IgM rheumatoid factors. Rheumatology, 2002;41(7):809-14).

Thus, serological testing of CCP IgG and RF IgM can miss a significant percentage of patients (so called sero-negative patients) who are developing rheumatoid arthritis and this could lead to a delay in diagnosis. Therefore, a need exists for means for detecting rheumatoid arthritis in a sero-negative individual to support diagnosis. An additional biomarker, which shows an elevated level in serum and/or plasma of early RA patients is anti-hnRNPB1/A2, also called RA33 (Hassfeld et a/., Demonstration of a new antinuclear antibody (anti-RA33) that is highly specific for rheumatoid arthritis. Arthritis Rheum, 1989; 32(12):1515-20). Additionally, because these serologic markers often become elevated, and damage to joints begins, up to several years before any onset of symptoms, many individuals experiencing early stages of rheumatoid arthritis are not even tested for the serologic markers until significant damage has occurred from disease progression. Finally, while it is recognized that there are multiple genetic factors associated with rheumatoid arthritis, the role and effect of such factors has not been elicited. Therefore, a need also exists for identifying individuals at risk, and particularly, at high risk, of developing rheumatoid arthritis so they can be adequately monitored and an early onset of rheumatoid arthritis can be diagnosed and effectively treated to prevent or delay joint damage and pain. There is also a need to provide further support in the diagnosis of rheumatoid arthritis.

### SUMMARY OF THE INVENTION

Accordingly, it is an object of the invention to provide methods, devices and systems which allow for or provide for improved diagnosis of rheumatoid arthritis and/or improved identification of individuals at risk of developing rheumatoid arthritis.

In this regard, the present invention is directed to a method for indicating a presence or absence of rheumatoid arthritis in an individual, said method comprising determining the presence or absence of one or more genetic variants, as defined herein, in a biological sample obtained from the individual, wherein the presence of at least one of the pairs of the genetic variants in the biological sample is indicative of the presence of rheumatoid arthritis in said individual as compared to the absence of all of the pairs of genetic variants in the biological sample. The present invention is also directed to a method for analyzing a biological sample from an individual for determining the presence of one or more genetic variants in the biological sample.

These methods comprise determining the presence of one or more of specific pairs of genetic variants in the biological sample of the individual. Each of the specific pairs of genetic variants have been discovered to be indicative of an elevated risk for the individual to develop RA and/or as being indicative of the presence of rheumatoid arthritis in said individual. In additional embodiments, the amounts or concentrations of additional biomarkers in a biological sample, for example, a serum and/or plasma sample, obtained from the individual are determined as well.

The present invention is also directed to a method of determining a level of risk for developing rheumatoid arthritis in an individual. The present invention is also directed to a computer-implemented method of determining a level of risk for developing rheumatoid arthritis in an individual, which method is executed by at least one processor in a computer.

These methods comprise determining the presence of one or more of specific pairs of genetic variants, as further defined herein, in the biological sample obtained from the individual, and correlating the presence of at least one of the pairs of genetic variants in the biological sample with an increased risk of the individual developing rheumatoid arthritis as compared with a risk of the individual developing rheumatoid arthritis in the absence of all of the pairs of genetic variants in the biological sample. Each of the specific pairs of genetic variants have been discovered to be indicative of an elevated risk for the individual to develop RA.

In additional embodiments, the amounts or concentrations of additional biomarkers in a biological sample, for example, a serum and/or plasma sample, from the individual and information regarding symptoms of clinical arthralgia in the individual are obtained as well, and the results are included in the correlation with the risk of developing rheumatoid arthritis.

The present invention is also directed to a panel, also referred herein to as an assay panel, of oligonucleotide primer pairs for multiplex polymerase chain reaction (PCR), or, in a specific embodiment, multiplex real-time polymerase chain reaction (real-time PCR), allowing amplification and identification of the specified genetic variants in a biological sample obtained from the individual. The genetic variants are members of specific pairs of genetic variants which have been discovered to be indicative of an elevated risk for the individual to develop RA.

Furthermore, the present invention is directed to systems, also referred to herein as assay systems. Such systems or assay systems may be used for determining rheumatoid arthritis markers in a biological sample. The systems or assay systems comprise a panel, or an assay panel, of oligonucleotide primer pairs as described herein and means for sequencing amplicons from a multiplex PCR conducted using the panel.

The present invention is also directed to a computer program product loadable into an internal memory of a computer, the computer program product comprising instructions for performing at least one correlating step of a method of determining a level of risk for developing rheumatoid arthritis in an individual as disclosed herein.

The present invention is also directed to the use of an assay panel or an assay system as described herein, in an *in vitro* method of diagnosis, prognosis or assessment of rheumatoid arthritis.

The methods, panels and systems of the invention are advantageous in allowing improved detection of rheumatoid arthritis, particularly in early stages and/or in seronegative individuals, and/or improved identification of an individual at risk of developing rheumatoid arthritis. Improved early detection allows for treatment that is more effective in preventing or slowing bone joint damage and identification of an individual at risk of developing rheumatoid arthritis may allow earlier detection and treatment of rheumatoid arthritis in an individual. These and additional objects and advantages will be more fully apparent in view of the detailed description.

### DETAILED DESCRIPTION

### Definitions

Details of the present invention are set forth below. Although any materials and methods similar or equivalent to those described herein can be used in the practice or testing of the present invention, the preferred materials and methods are now described.

Other features, objects and advantages of the invention will be apparent from the description. In the description, the singular forms also include the plural unless the context clearly dictates otherwise.

Unless defined otherwise, all technical and scientific terms used herein have the same meaning as commonly understood by one of ordinary skill in the art to which this invention belongs.

The term "biological sample" as used herein includes any biological specimen obtained from a subject suitable for the intended purpose. The skilled person is aware of which biological sample to use depending on the technology used. Examples of biological samples are described elsewhere herein.

The terms "individual" "subject" or "patient" may be referred to interchangeably herein and in reference to a human patient.

To "indicate" or "indicating" a presence of rheumatoid arthritis in an individual as referred to in a method disclosed herein does not mean that the individual will be attributed to a clinical picture through such a method but rather that such an indication may be used in combination with other measures to clarify a pathological situation. The terms "diagnosis" or "diagnostic", or similar expressions are sometimes used herein. Notably, a particular diagnostic method need not provide a definitive diagnosis of a condition but rather provide a positive indication that aids or assists in a diagnosis. As an example, it may form part of establishing a diagnostic or clinical picture, but it need not in itself lead to a full diagnosis.

The terms "prognosis" or "determining a predisposition" of a condition refers to a forecast as to the probable outcome of a condition and sometimes to the prospect of recovery from a condition as indicated by the nature and symptoms of the case. Herein, it is sometimes also referred to the "assessment" of rheumatoid arthritits. In this regard the term assessment refers broadly to any evaluation of the condition or of a predisposition to develop such a condition. Other similar terms may also be used in this context and are known to the skilled person.

The method herein disclosed is a non-invasive, *ex-vivo* or *in-vitro* method. If the herein described methods are non-invasive, the term "providing a biological" sample shall not be interpreted to mean that the method of the present disclosure includes a surgical procedure conducted on the subject. Accordingly, the method of the present disclosure is an *in vitro* method in the sense that the biological sample or the serum and/or plasma sample referred to herein has previously been obtained from the individual, thereby not necessitating the presence of the individual when performing the herein disclosed method. Hence, it to be understood that a method as disclosed herein does not encompass any invasive procedure on the human body.

The term "single nucleotide polymorphisms" (SNP) refer to the genetic properties of a defined locus in the genetic code of an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The Single Nucleotide Polymorphism Database (dbSNP) is an archive for genetic variation within and across different species developed and hosted by the National Center for Biotechnology Information (NCBI) in collaboration with the National Human Genome Research Institute (NHGRI), both located in the US. Although the name of the database implies a collection of one class of polymorphisms only (i.e., single nucleotide polymorphisms (SNP)), it in fact contains a range of molecular variation. Every unique submitted SNP record receives a reference SNP ID number ("rs#"; "refSNP cluster"). Herein, SNP are mainly identified using rs# numbers.

A "biomarker" or "marker" in the context of the present disclosure refers to a protein which is differentially present in a biological sample taken from individuals having a certain condition as compared to a comparable sample taken from individuals who do not have said condition (e.g., negative diagnosis, normal or healthy individual). "Differentially present" in this regard may for example be that the biomarker is present in a higher or lower concentration in a biological sample obtained from an individual suffering from a condition, as compared to a biological sample obtained from a healthy individual.

Herein, it is referred to "determining a level of risk" of rheumatoid arthritis and determining that an individual has an "increased risk" of developing rheumatoid arthritis based on the presence of a set of genetic markers disclosed herein. There is no set percentage value associated with an "increased risk" but an individual who has at least one pair of the genetic variants disclosed herein will be more exposed to the condition than an individual who does not possess any of the genetic variants.

Accordingly, the methods, devices and systems of the invention are derived from the discovery that the presence of certain pairs of genetic variants are indicative of an increased risk of developing rheumatoid arthritis. The genetic variants are identified by their respective dbSNP Reference SNP ("rs") numbers (see www.ncbi.nlm.nih.gov/snp/docs/RefSNP_about/). Generally, variants comprise small variations (less than about 50 base pairs) in a gene and typically comprise a single nucleotide variation, short multi-nucleotide variations, small deletions or insertions, small short tandem repeats (STRs), or retrotransposable element insertions. The pairs of genetic variants employed in the present invention are as follows:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270.

The associated genes and pathways of the indicated variants (as provided by Gene Card - the Human Gene Database, genecards.org; OMIM^{®} - Online Mendelian Inheritance in Man^{®}, omim.org; or NCBI, ncbi.nlm.nih.gov) are as follows:

| rs number | Gene | Pathway |
|---|---|---|
| rs1045642 | ABCB1 | Pharmacodynamics |
| rs2298383 | ADORA2A | Signaling by G-protein-coupled receptors (GPCR) |
| rs4700060 | ANKRD55 | Nuclear factor kappa B (NF-κB) and Notch transcription pathways |
| rs4586 | CCL2 | Folate Metabolism |
| rs11574914 | CCL21 | Signaling by GPCR |
| rs4810485 | CD40 | transcriptional misregulation in cancer |
| rs25882 | CSF2 | RET signaling |
| rs3027898 | IRAK1 | Nod-like receptor (NLR) signaling pathway |
| rs17367504 | MTHFR | Folate Metabolism |
| rs17375901 | MTHFR | Folate Metabolism |
| rs2240340 | PADI4 | protein citrullination |
| rs874881 | PADI4 | protein citrullination |
| rs2847297 | PTPN2 | Signaling by GPCR |
| rs7403531 | RASGRP1 | RET signaling; Signaling by GPCR |
| rs387907270 | RBPJ | Signaling by GPCR |
| rs2014300 | RUNX1 | transcriptional misregulation in cancer |
| rs1050152 | SLC22A4 | Phospholipase D signaling |
| rs10181656 | STAT4 | GPCR Pathway |
| rs11889341 | STAT4 | GPCR Pathway |
| rs3821236 | STAT4 | GPCR Pathway |
| rs1800629 | TNF | Apoptosis Modulation an Signaling |

More specifically, the variants are described by the following, based on the human genome database hg38, reference GRCh38.p2:

| Chromosome | Start | End | rs number | Sequence |
|---|---|---|---|---|
| chr7 | 87509328 | 87509329 | rs1045642 | REF=A;OBS=G |
| chr22 | 24429542 | 24429543 | rs2298383 | REF=C;OBS=A |
| chr5 | 56214828 | 56214829 | rs4700060 | REF=C;OBS=T |
| chr17 | 34256249 | 34256250 | rs4586 | REF=T;OBS=C |
| chr9 | 34710340 | 34710341 | rs11574914 | REF=G;OBS=A |
| chr20 | 46119307 | 46119308 | rs4810485 | REF=T;OBS=G |
| chr5 | 132075766 | 132075767 | rs25882 | REF=T;OBS=C |
| chrX | 154010438 | 154010439 | rs3027898 | REF=C;OBS=A |
| chr1 | 11802720 | 11802721 | rs17367504 | REF=A;OBS=G |
| chr1 | 11792458 | 11792459 | rs17375901 | REF=C;OBS=T |
| chr1 | 17336143 | 17336144 | rs2240340 | REF=T;OBS=C |
| chr1 | 17334003 | 17334004 | rs874881 | REF=G;OBS=C |
| chr18 | 12797694 | 12797695 | rs2847297 | REF=A;OBS=G |
| chr15 | 38530703 | 38530704 | rs7403531 | REF=T;OBS=C |
| chr4 | 26406263 | 26406264 | rs387907270 | REF=A;OBS=G |
| chr21 | 34985563 | 34985564 | rs2014300 | REF=A;OBS=G |
| chr5 | 132340626 | 132340627 | rs1050152 | REF=C;OBS=T |
| chr2 | 191105152 | 191105153 | rs10181656 | REF=G;OBS=C |
| chr2 | 191079015 | 191079016 | rs11889341 | REF=C;OBS=T |
| chr2 | 191038031 | 191038032 | rs3821236 | REF=G;OBS=A |
| chr6 | 31575253 | 31575254 | rs1800629 | REF=G;OBS=A |

Above, "REF" (Reference) means that the general population possesses the nucleotide in the position referred to and "OBS" (Observed) means the single nucleotide polymorphism present in this position.

Accordingly, based on the herein disclosed findings of specific genetic variants related to rheumatoid arthritis, there is provided a method for indicating a presence or absence of rheumatoid arthritis in an individual. The method comprises determining the presence in the biological sample obtained from the individual of one or more of the following pairs of genetic variants:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270, wherein the presence of at least one of the pairs of the genetic variants in the biological sample is indicative of the presence of rheumatoid arthritis in said individual as compared to the absence of all of the pairs of genetic variants in the biological sample. The method as disclosed herein is also useful for aiding or assisting in a diagnosis or prognosis of rheumatoid arthritis.

The present invention is also directed to a method for analyzing a biological sample from an individual. The method comprises determining the presence in the biological sample of one or more of the pairs of genetic variants as disclosed herein.

While the individual genetic variants included in these specified pairs may have previously been associated with one or more diseases, including but not limited to rheumatoid arthritis, it has now been discovered that the indicated specific pairs of variants are indicative of an increased risk of an individual developing rheumatoid arthritis. Specifically, using populations of rheumatoid arthritis individuals, healthy individuals, rheumatoid arthritis seronegative individuals, rheumatoid arthritis seropositive individuals, and disease control individuals, screening of about 310 genetic variants within about 70 genes was conducted using NGS (next generation sequencing) AmpliSeq^{™} technology on the Ion GeneStudio^{™} instruments (Thermo Fisher Scientific) performing a targeted sequencing approach. Identification of the 310 genetic variants included in the approach was based on specific data base research using, inter alia, DisGeNET, CTD, CLINVAR, and ORPHANET, and various scoring techniques. The results were analyzed to arrive at the indicated pairs as representative of an increased risk of developing rheumatoid arthritis, particularly as compared with a risk of an individual developing rheumatoid arthritis in the absence of all of the pairs of genetic variants in a biological sample from the individual.

The biological sample may be any biological sample that provides sufficient genetic material, i.e., DNA, for example, hair, cell tissue, saliva, blood, or the like, for determining the presence of the indicated genetic variant pairs. In a specific embodiment, the biological sample is saliva or a whole blood sample, and in a more specific embodiment, the sample is a whole blood sample. As mentioned elsewhere herein, the biological sample has previously been obtained from the individual, i.e., the step of obtaining the biological sample from the individual is not part of the herein disclosed method.

Various techniques are known in the art for determining the presence or absence of a specific genetic variant in a biological sample, or, specifically saliva or a whole blood sample, and one or more of such known techniques may be employed herein. In a specific embodiment, polymerase chain reaction (PCR) techniques may be employed, and conveniently, real time PCR, or more specifically, real time multiplex PCR, using oligonucleotide primer pairs allowing amplification of the genetic variants of the indicated pairs of interest and identification of amplified variants, may be employed. Alternatively, or in addition, other sequencing technologies may be employed, including, but not limited to, Next Generation Sequencing (NGS) and/or Sanger Sequencing.

The individual may be from the general population, or in a specific embodiment, may be a member of a select population. For example, the individual may be a first degree relative (sibling, parent, child) of a previously-diagnosed rheumatoid arthritis patient. First degree relatives are at a higher risk of developing rheumatoid arthritis as compared with the general population (MacGregor, et al., Characterizing the quantitative genetic contribution to rheumatoid arthritis using data from twins. Arthritis & Rheumatism, 2000. 43(1): p. 30-37). Alternatively, or in addition, the individual may belong to a population identified as having a higher risk of developing rheumatoid arthritis, namely, being of female gender, tobacco smokers, and/or individuals exhibiting obesity. In additional embodiments, the individual may have previously been subjected to serological testing for CCP IgG and/or RF IgM and determined to be serologically positive, i.e., one or both markers having been detected in a serum and/or plasma sample of the individual, or serologically negative, i.e., neither of the markers having been detected in a serum and/or plasma sample of the individual. In an additional embodiment, the individual may be asymptomatic or may be exhibiting one or more symptoms of arthritis, for example, arthralgia, i.e., joint pain, as exhibited by joint symptoms of a duration of less than one year, symptoms located in the metacarpophalangeal (MCP) joints, morning stiffness for greater than 60 minutes, severe morning symptoms, difficulty of making a fist, and/or a positive squeeze test of the MCP joints (Aletaha et al., 2010 Rheumatoid arthritis classification criteria: an American College of Rheumatology/European League Against Rheumatism collaborative initiative, Ann Rheum Dis 2010;69:1580-1588).

In additional embodiments of the method, further analysis of biological markers is conducted in combination with the analysis of the genetic markers as mentioned elsewhere herein. In one specific embodiment, serologic analysis of a blood sample, or, more specifically, plasma and/or serum, of the individual is conducted. In a specific embodiment, the sample is a serum sample or a plasma sample. An analysis of biological markers comprises measuring or determining a concentration or an amount of one of more biomarkers. Herein, the terms "biomarkers" and "biological markers" may be used interchangeably.

Accordingly, in one embodiment, a method as disclosed herein comprises determining a concentration in a serum and/or plasma sample obtained from the individual of one or more of the biomarkers: anti-cyclic citrullinated peptide immunoglobulin G (CCP IgG), rheumatoid factor immunoglobulin M (RF IgM), anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and/or anti-rheumatoid arthritis 33 immunoglobulin M (RA33 IgM). The presence of at least one of the biomarkers in a concentration level above its respective upper limit of normal in the serum and/or plasma sample is indicative of the presence of rheumatoid arthritis in said individual.

For example, the respective concentrations of the biomarkers CCP IgG and RF IgM in a serum and/or plasma sample obtained from the individual may be determined. In additional embodiments, the respective concentrations of one or more, or all, of the biomarkers anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM) in a serum and/or plasma sample obtained from the individual may be determined. In a specific embodiment, the respective concentrations of the biomarkers CCP IgG, CCP IgA, RF IgM, and RF IgA are determined, and optionally, the respective concentrations of the biomarkers RA33 IgA, RA33 IgG, and/or RA33 IgM are determined. Sometimes, it is also referred to herein to measure an amount of one or more of the herein disclosed biomarkers, and/or comparing amounts of biomarkers to an upper limit of normal in the serum and/or plasma sample of the biomarker.

Within the present disclosure, reference to the determination of an amount of a biomarker refers to a qualitative or quantitative determination of an amount relative to the respective upper limit of normal for the biomarker. In a method of the present disclosure, it may herein also be referred to measuring or determining a concentration of a biomarker or determining or measuring a concentration level of a biomarker relative to the respective upper limit of normal for the biomarker. Generally, the upper limit of normal for a particular biomarker is specified in the directions for use (DFU) accompanying a diagnostic device for the individual biomarker. Each DFU indicates the defined ranges "negative", "equivocal" and "positive" for each marker, with the positive range starting at the upper limit of normal. For example, the Phadia EliA^{®} fluoroenzyme immunoassay for CCP indicates an upper limit of normal as 10 U/ml for each of CCP IgG and CCP IgA, while the Phadia EliA^{®} fluoroenzyme immunoassays for RF IgM and RF IgA indicate upper limits of normal as 5 IU/ml and 20 IU/ml, respectively.

The respective amounts or concentrations of the biomarkers CCP IgG, CCP IgA, RF IgM, RF IgA, RA33 IgA, RA33 IgG, and/or RA33 IgM may be determined using any of the various techniques known in the art for determining the amount or concentration of an immunoglobulin biomarker in a biological sample, or, specifically a serum and/or plasma sample. In a specific embodiment, the amounts of these biomarkers are determined via immunoassay, including, but not limited to radioimmunoassay (RIA), enzyme-linked immunosorbent assay (ELISA), for example, a fluorescence enzyme immunoassay or fluorimetric enzyme-linked immunoassay (FEIA). In a more specific embodiment, the immunoassay employs one or more solid phases having immobilized respective proteins and/or antigens as targets for the biomarkers. In another embodiment, the immunoassay employs a liquid solution. In further embodiments, a microarray may be used for determining the amount of an immunoglobulin biomarker in a biological sample.

As one non-limiting example, CCP biomarkers may be determined using the Phadia EliA^{®} fluoroenzyme immunoassay. In this immunoassay, the EliA^{®} wells are coated with citrullinated synthetic peptides. If present in the serum and/or plasma sample, antibodies to CCP bind to their specific antigen. After washing away non-bound antibodies, enzyme-labeled antibodies against human IgG antibodies (EliA^{®} IgG conjugate) are added to form an antibody-conjugate complex. After incubation, non-bound conjugate is washed away and the bound complex is incubated with a development solution. After stopping the reaction, the fluorescence in the reaction mixture is measured. The higher the response value, the more specific IgG is present in the specimen. To evaluate test results, the response for the serum and/or plasma sample is compared directly to the response for calibrators. Similar EliA^{®} immunoassays may be used for determining RF IgM and RF IgA. One of ordinary skill in the art will appreciate that other methods and devices may be used for determining amounts of such biomarkers in a serum and/or plasma sample.

A method as disclosed herein may also further comprise obtaining information regarding symptoms of arthralgia in said individual, as described elsewhere herein. In this regard, the terms "obtaining information" or "collecting information" may be used interchangeably.

As the presence of one or more pairs of genetic variants represents an increased or higher risk of an individual developing rheumatoid arthritis, the present invention is also directed to a computer-implemented method of determining a level of risk for developing rheumatoid arthritis in an individual, based on the presence of the one or more pairs of genetic variants in the individual, which method is executed by at least one processor in a computer.

The present invention also concerns a method of determining a level of risk, based on the presence of the one or more pairs of genetic variants in the individual, for developing rheumatoid arthritis in an individual.

Such methods comprise determining the presence or absence of one or more of the indicated pairs of genetic variants as disclosed elsewhere herein in a biological sample obtained from the individual, and correlating the presence of at least one of the pairs of genetic variants in the biological sample with an increased risk of the individual developing rheumatoid arthritis as compared with the risk of the individual developing rheumatoid arthritis in the absence of all of the pairs of genetic variants in the biological sample. When an individual is found to have such an increased risk of developing rheumatoid arthritis, the individual is a candidate for more frequent serologic testing for relevant biomarkers, for example, for CCP IgG and/or RF IgM, and/or more targeted evaluation of any arthritis-associated symptoms, in order to increase the likelihood of early detection and treatment of rheumatoid arthritis.

In more specific embodiments of a method of determining a level of risk for developing rheumatoid arthritis in an individual, such as a computer-implemented method disclosed herein, additional information from the individual is obtained and included in the risk correlation. For example, in one embodiment, the method further comprises determining the concentrations in a serum and/or plasma sample from the individual of the biomarkers CCP IgG and RF IgM, and obtaining information regarding symptoms of arthralgia in said individual. The method then comprises correlating at least two of (a) the presence in the biological sample of at least one of the pairs of genetic variants, (b) the presence in the serum and/or plasma sample of at least one of the biomarkers in a concentration level above its respective upper limit of normal, and (c) the presence of symptoms of arthralgia, with an increased risk of the individual developing rheumatoid arthritis as compared with the risk of the individual developing rheumatoid arthritis in the absence of at least two of (a)-(c). Thus, the serologic information and/or symptom information are combined with the genetic variant pair information to provide an improved determination of the level of risk for developing rheumatoid arthritis in the individual.

In a further embodiment, in the absence of (a) and (c), i.e., at least one pair of genetic variants was not detected in the biological sample, and the individual is not experiencing any symptoms of arthralgia, the method further comprises correlating (i) the presence in the serum and/or plasma sample of both of the biomarkers CCP IgG and RF IgM in concentration levels above their respective upper limits of normal, and/or (ii) the presence of CCP IgG at a concentration level more than three times its upper limit of normal, and/or (iii) the presence of RF IgM at a concentration level more than ten times its upper level of normal with an increased risk of the individual developing rheumatoid arthritis as compared with the risk of the individual developing rheumatoid arthritis in the absence of (i), (ii) and (iii)

In further embodiments, in the absence of (a) and (c), i.e., at least one pair of genetic variants was not detected in the biological sample, and the individual is not experiencing any symptoms of arthralgia, in addition to the determination of the concentrations of the biomarkers CCP IgG and RF IgM in a serum and/or plasma sample obtained from the individual, the concentrations of one or more, or all, of the biomarkers CCP IgA, RF IgA, RA33 IgA, RA33 IgG and RA33 IgM in a serum and/or plasma sample obtained from the individual are determined. The results of these determinations are correlated with the risk of developing rheumatoid arthritis. Specifically, in this case, the presence of two or more of the biomarkers CCP IgG, RF IgM, CCP IgA, RF IgA, RA33 IgA, RA33 IgG and RA33 IgM in the serum and/or plasma sample in concentration levels above their respective upper limits of normal is correlated with an increased risk of the individual developing rheumatoid arthritis as compared with the risk of the individual developing rheumatoid arthritis if at least two biomarkers do not reach concentration levels above the upper limit of normal in the serum and/or plasma sample, i.e., in the absence of at least two of the biomarkers concentration levels above their upper limits of normal.

In further embodiments, in the absence of (a) and (c), i.e., at least one pair of genetic variants was not detected in the biological sample, and the individual is not experiencing any symptoms of arthralgia, the concentrations of CCP IgG and RF IgM, and the concentration of at least one biomarker, or all the biomarkers, selected from CCP IgA, RF IgA, RA33 IgA, RA33 IgG and RA33 IgM in a serum and/or plasma sample obtained from the individual are determined. The results of these determinations are correlated with the risk of developing rheumatoid arthritis. Specifically, in this case, (i) the presence of at least one biomarker selected from CCP IgG, CCP IgA, RF IgA, RA33 IgA, RA33 IgG and RA33 IgM at a concentration level more than three times its respective upper limit of normal, also referred to herein as a high positivity level, and/or (ii) the presence of RF IgM at a concentration level more than ten times its upper limit of normal, also referred to herein as a high positivity level, is correlated with an increased risk of the individual developing rheumatoid arthritis as compared with the risk of the individual developing rheumatoid arthritis in the absence of at least one of the biomarkers at such a concentration level (also referred to as a high positivity level) in the serum and/or plasma sample obtained from the individual.

In this regard, when a method disclosed herein refers to the individual not experiencing any symptoms of arthralgia, this means that information had previously been obtained or collected that said individual had not experienced any symptoms of arthralgia.

In any of the aforementioned methods of determining a risk of an individual to develop rheumatoid arthritis, the correlation step may also take into account information regarding the individual's predisposition in a high risk group, for example being a first degree relative (sibling, parent, child) of a person previously diagnosed as having rheumatoid arthritis, the presence of obesity, the gender of the individual being female, and/or the individual's regular smoking of tobacco. Accordingly, the methods disclosed herein may further comprise obtaining information regarding one or more of the following factors: the presence of obesity in said individual, the gender of the individual being female and the individual's regular smoking of tobacco, wherein the presence of one or more of said factors correlates to an increased risk of the individual developing rheumatoid arthritis as compared to a risk of the individual in developing rheumatoid arthritis in the absence of all of the factors. This may also be referred to as obtaining information regarding if one or more of the conditions are fulfilled for the individual mentioned herein.

In any of the aforementioned methods employing combinations of different data, the combination of data can be combined and assessed according to any kind of algorithmic combination of results, including, for example, a linear combination of data or a combination employing a non-linear polynomial relationship. Other possible methods for combining data into a model capable of producing a risk estimate include (but are not limited to) non-linear polynomials, support vector machines, neural network classifiers, discriminant analysis, random forest, gradient boosting, partial least squares, ridge regression, lasso, elastic nets, and k-nearest neighbors. Furthermore, the book "The Elements of Statistical Learning: Data Mining, Inference, and Prediction, Second Edition" by T Hastie, R Tibshirani and J Friedman as published by Springer Series in Statistics, ISBN 978-0387848570 (which is incorporated by reference herein) describes many suitable methods for combining the determined data in order to predict or classify a particular outcome or, for example, to assign a risk score.

The present invention is also directed to devices and systems for conducting one or more steps in the methods of the invention. More specifically, the invention is directed to an assay system and an assay panel. Hence, there is provided herein a panel, or an assay panel, of oligonucleotide primer pairs for multiplex polymerase chain reaction (PCR) allowing amplification and identification of the following genes in a DNA-containing sample: rs1050152, rs3821236, rs1800629, rs25882, rs17367504, rs3027898, rs11574914, rs7403531, rs11889341, rs874881, rs2240340, rs4586, rs2014300, rs4700060, rs2847297, rs4810485, rs10181656, rs17375901, rs1045642, rs2298383 and rs387907270. A "panel" of primer pairs in this regard is intended to mean a set, or a number of nucleic acid primers that are used for identifying the genes in a DNA-containing sample. In a specific embodiment, the panel, or assay panel of oligonucleotide primers is provided on a genotyping array.

There is also provide herein a kit or a kit of parts comprising oligonucleotide primer pairs for multiplex polymerase chain reaction (PCR) allowing identification of the genes in a DNA-containing sample as described herein. Such a kit or kit of parts may also include other means for performing the multiplex polymerase chain reaction, such as buffers and solutions well-known to a person skilled in the art for performing such reactions, optionally in combination with instructions for use.

Devices for performing the methods of the present disclosure are also provided herein. In this regard, there is also provided a device comprising an assay panel comprising oligonucleotide primers as described herein. A device may in this regard constitute automated means for performing the multiplex polymerase chain reaction, which means are well-known to a person skilled in the art.

Furthermore, the present invention is also directed to a system, or an assay system, wherein said assay system may be used for determining rheumatoid arthritis markers in a biological sample. The assay system comprises the assay panel of oligonucleotide primer pairs as described above for multiplex PCR and means for sequencing amplicons from a multiplex PCR conducted using the panel. The means for sequencing amplicons may also be referred to as a "sequencer" and is an automated means, device or apparatus, adapted to perform these types of reactions. Such means are well-known to the skilled person. The system may further optionally include a computer program product, for example, a computer program product loadable into an internal memory of a computer, the computer program product comprising instructions for correlating the presence of one or more of the specified pairs of genetic variants in sequenced amplicons from a multiplex PCR to a risk of developing rheumatoid arthritis. Such a computer program product may further include instructions for correlating any of the aforementioned combinations of data to a risk of developing rheumatoid arthritis.

Therefore, in yet a further embodiment, the invention is directed to a computer program product loadable into an internal memory of a digital computer. The computer program product comprises instructions for performing at least one of the correlating steps of any of the methods as described herein. The computer program product is adapted to perform at least one of the correlating steps when executed in a computer comprising at least one processor and memory.

There is also provided herein a cloud-based software for performing at least one of the correlating steps of a method according to the present disclosure.

There is also provided herein the use of an assay panel comprising oligonucleotide primers or an assay system as described herein, in an *in vitro* method of diagnosis, prognosis or assessment, such as risk assessment, of rheumatoid arthritis.

### Itemized embodiments

Embodiment 1: A method for analyzing a biological sample from an individual, comprising determining the presence of one or more of the following pairs of genetic variants in the biological sample:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270.

Embodiment 2: The method of embodiment 1, further comprising determining the respective amounts in a serum and/or plasma sample from the individual of the biomarkers: anti-cyclic citrullinated peptide immunoglobulin G (CCP IgG) and rheumatoid factor immunoglobulin M (RF IgM).

Embodiment 3: The method of embodiment 2, further comprising determining the respective amounts in a serum and/or plasma sample from the individual of one or more of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM).

Embodiment 4: The method of embodiment 3, further comprising determining the respective amounts in a serum and/or plasma sample from the individual of all of the biomarkers: CCP IgA, RF IgA, RA33 IgA, RA33 IgG, and RA33 IgM.

Embodiment 5: The method of embodiment 1, further comprising determining the respective amounts in a serum and/or plasma sample from the individual of the biomarkers: anti-cyclic citrullinated peptide immunoglobulin G (CCP IgG), anti-cyclic citrullinated peptide immunoglobulin A (CCP IgA), rheumatoid factor immunoglobulin M (RF IgM), and rheumatoid factor immunoglobulin A (RF IgA).

Embodiment 6: The method of embodiment 5, further comprising determining the respective amounts in a serum and/or plasma sample from the individual of the biomarkers: anti-rheumatoid arthritis 33 immunoglobulin A (RA33 IgA), anti-rheumatoid arthritis 33 immunoglobulin G (RA33 IgG), and/or anti-rheumatoid arthritis 33 immunoglobulin M (RA33 IgM).

Embodiment 7: The method of any one of embodiments 1-6, further comprising determining if the individual exhibits arthralgia.

Embodiment 8. A method of determining a level of risk for developing rheumatoid arthritis in an individual, the method comprising: determining the presence or absence in a biological sample from the individual of one or more of the following pairs of genetic variants:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270; and
correlating the presence of at least one of the pairs of genetic variants in the biological sample with an increased risk of the individual developing rheumatoid arthritis as compared with a risk of the individual developing rheumatoid arthritis in the absence of all of the pairs of genetic variants in the biological sample.

Embodiment 9. The method of embodiment 8, further comprising
determining the relative amounts in a serum and/or plasma sample from the individual of the biomarkers: anti-cyclic citrullinated peptide immunoglobulin G (CCP IgG) and rheumatoid factor immunoglobulin M (RF IgM);
determining if the individual exhibits arthralgia; and
correlating at least two of (a) the presence of at least one of the pairs of genetic variants in the biological sample, (b) the presence of at least one of the biomarkers in an amount above its respective upper limit of normal in the serum and/or plasma sample, and (c) the presence of arthralgia, with an increased risk of the individual developing rheumatoid arthritis as compared with the risk of the individual developing rheumatoid arthritis in the absence of at least two of (a)-(c).

Embodiment 10: The method of embodiment 9, in the absence of (a) and (c), correlating (i) the presence of both of the biomarkers in amounts above their respective upper limits of normal, and/or (ii) the presence CCP IgG at a level more than three times its upper limit of normal, and/or (iii) the presence of RF IgM at a level more than ten times its upper level of normal with an increased risk of the individual developing rheumatoid arthritis as compared with the risk of the individual developing rheumatoid arthritis in the absence of (i), (ii) and (iii).

Embodiment 11: The method of embodiment 9, in the absence of (a) and (c), further comprising determining the respective amounts of one or more of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM) in a serum and/or plasma sample from the individual; and
correlating the presence of two or more of the biomarkers CCP IgG, RF IgM, CCP IgA, RF IgA, RA33 IgA, RA33 IgG and RA33 IgM in the serum and/or plasma sample in amounts above their respective upper limits of normal with an increased risk of the individual developing rheumatoid arthritis as compared with the risk of the individual developing rheumatoid arthritis in the absence of at least two of the biomarkers in an amounts above their respective upper limits of normal in the serum and/or plasma sample.

Embodiment 12: The method of embodiment 9, in the absence of (a) and (c), further comprising determining the respective amounts of one or more of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM) in a serum and/or plasma sample from the individual; and
correlating (i) the presence of at least one biomarker selected from CCP IgG, CCP IgA, RF IgA, RA33 IgA, RA33 IgG and RA33 IgM at a level more than three times its respective upper limit of normal, and/or (ii) the presence of RF IgM at a level more than ten times its upper level of normal with an increased risk of the individual developing rheumatoid arthritis as compared with the risk of the individual developing rheumatoid arthritis in the absence (i) and (ii).

Embodiment 13: The method of any one of embodiments 8-12, wherein the presence of one or more of the following factors correlates to an increased risk of the individual developing rheumatoid arthritis as compared to a risk of the individual in developing rheumatoid arthritis in the absence of all of the following factors: the presence of obesity, the gender of the individual being female, and the individual's regular smoking of tobacco.

Embodiment 14: The method of embodiment 1 or embodiment 8, wherein the individual has previously tested negative for the presence of the biomarker anti-cyclic citrullinated peptide immunoglobulin G (CCP IgG) in a serum and/or plasma sample in an amount above its upper limit of normal.

Embodiment 15: The method of any one of embodiments 1, 8 or 14, wherein the individual has previously tested negative for the presence of the biomarker rheumatoid factor immunoglobulin M (RF IgM) in a serum and/or plasma sample in an amount above its upper limit of normal.

Embodiment 16: The method of any one of embodiments 1-15, wherein the individual is a first degree relative of a person previously diagnosed with rheumatoid arthritis.

Embodiment 17: The method of any one of embodiments 1-16, wherein the presence of at least one of the pairs of genetic variants is determined using polymerase chain reactions.

Embodiment 18: The method of any one of embodiments 2-7 and 10-12, wherein the presence and/or amount of the biomarkers is determined via immunoassay or, more specifically, fluorescence enzyme immunoassay.

Embodiment 19: The method of any one of embodiments 2-7 and 10-12, wherein the presence and/or amount of the biomarkers is determined with a microarray.

Embodiment 20: The method of any one of embodiments 1-19, wherein the biological sample comprises a whole blood sample.

Embodiment 21: A panel of oligonucleotide primer pairs for multiplex polymerase chain reaction (PCR) allowing amplification and identification of the following genes in a biological sample:
rs1050152, rs3821236, rs1800629, rs25882, rs17367504, rs3027898, rs11574914, rs7403531, rs11889341, rs874881, rs2240340, rs4586, rs2014300, rs4700060, rs2847297, rs4810485, rs10181656, rs17375901, rs1045642, rs2298383 and rs387907270.

Embodiment 22. The panel of embodiment 21, provided on a genotyping array.

Embodiment 23. A system for determining rheumatoid arthritis markers in a biological sample, comprising the panel of embodiment 21 or 22, and means for sequencing amplicons from a multiplex PCR conducted using the panel.

Embodiment 24. The system of embodiment 23, further comprising a computer program directly loadable into the internal memory of a digital computer, the computer program product comprising software code means for correlating the presence of one or more of the following pairs of genetic variants in the sequenced amplicons to a risk of developing rheumatoid arthritis:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270.

Embodiment 25. A computer program product directly loadable into the internal memory of a digital computer, the computer program product comprising software code means for performing the correlating step of the method of any one of embodiments 8-13.

The following examples demonstrate various aspects of the methods of the invention, nevertheless, without being limited thereto.

### EXPERIMENTAL SECTION

### EXAMPLE 1

This example describes various embodiments of the methods of the invention and employed a study population of 1227 first degree relatives of rheumatoid arthritis patents (FDR-RAs) from the Swiss multicenter cohort study SCREEN-RA. Genetic analysis of genomic DNA isolated from whole blood samples from the study population was conducted using the next generation sequencing (NGS) technology AmpliSeq^{™} on the Ion GeneStudio^{™} instruments from Thermo Fisher Scientific, USA, to determine the presence or absence of the indicated 14 pairs of genetic variants. Using serum samples and the EliA^{™} technology (Phadia AB, Sweden), anti-CCP IgG/IgA (CE-IVD) and RF IgM/IgA (CE-IVD) were measured. Anti-RA33 IgM/IgA/IgG (RUO, research use only) in serum samples was also measured using the EliA^{™} technology. Serologic data measured using commercially available CE-IVD tests from the mentioned biomarkers can be combined with the genetic information. Clinical arthralgia information for individual subjects was provided with the serum samples. Specifically, clinically suspected arthralgia (CSA) was determined using the EULAR (European League against Rheumatism) definition published in 2016 and the recommended symptoms and questions were included (van Steenbergen et al., Annals of the Rheumatic Diseases, 2017; 76:491-496). A score was estimated for each criterion (clinical arthralgia information, protein serology, and genetic variant pair) and combined into an individual overall risk score. Specifically, to combine the serology, genetic and CSA datasets, a positive outcome was defined for each of the datasets. For the serology, positivity for at least one included marker, i.e., anti-CCP IgG/IgA (CE-IVD), RF IgM/IgA (CE-IVD), or anti-RA33 IgM/IgA/IgG (RUO, research use only) was sufficient to be positive. For the genetics, detection of one or more of the indicated 14 pairs was needed to be positive. For CSA, a score greater or equal to 4 was needed to be positive.

Eighty individuals were identified as positive for at least one of the indicated pairs of genetic variants, with the presence of at least one such pair correlating to an increased risk of developing rheumatoid arthritis. Additionally, 103 individuals were identified as positive for at least one of the protein biomarkers CCP IgG, RF IgM, CCP IgA, RF IgA, RA33 IgA, RA33 IgG and RA33 IgM, and 89 individuals showed clinical arthralgia with a CSA score greater or equal 4.

In a first step of a more detailed two-step evaluation of risk, of these individuals meeting at least one criteria of genetic variants, serologic biomarkers and clinical arthralgia, those meeting at least two of the criteria were correlated with a high risk of developing rheumatoid arthritis. Specifically, 11 individuals were identified as positive for at least one of the indicated pairs of genetic variants and as positive for at least one of the serologic biomarkers, 6 individuals were identified as positive for at least one of the indicated pairs of genetic variants and as showing clinical arthralgia, and 31 individuals were identified as positive for at least one of the serologic biomarkers and as showing clinical arthralgia. Three individuals were identified as positive for at least one of the indicated pairs of genetic variants, as positive for at least one of the serologic biomarkers, and as showing clinical arthralgia. Therefore, for 51 individuals, a high risk of developing rheumatoid arthritis correlated with meeting at least two of the three criteria of genetics, serology, and clinical arthralgia.

Of the 103 individuals identified as positive for at least one of the serologic biomarkers, 58 individuals did not show either a specified pair of the genetic variants or clinical arthralgia. A second step of analysis was therefore conducted with respect to these 58 individuals. In this second step, 35 individuals were identified as uniquely positive for serology in that they either showed positivity to two or more of the serologic biomarkers and/or a high positivity (above 3 times the upper limit of normal for CCP IgG, CCP IgA, RF IgA, RA33 IgA, RA33 IgG or RA33 IgM and/or 10 times the upper limit of normal for RF IgM), and therefore also correlated with a high risk of developing rheumatoid arthritis, despite the absence of the genetic variants or clinical arthralgia.

Among the Screen-RA cohort, 13 individuals have been subsequently diagnosed with clinical rheumatoid arthritis. Nine of the 13 (70%) were identified among the 86 individuals identified as having a high risk (51 individuals meeting at least two of the three criteria of genetics, serology, and clinical arthralgia, and 35 individuals not meeting at least two of the three criteria but instead identified as uniquely positive for serology).

Identification of individuals at risk for developing rheumatoid arthritis provides a basis for more closely monitoring such individuals to avoid joint damage often encountered prior to diagnosis based only on clinical symptoms, allowing early treatment with disease-modifying antirheumatic drugs.

### EXAMPLE 2

This example describes various embodiments of the methods of the invention and employed a study population of 4138 rheumatoid arthritis patients. Using the genetic analysis techniques described in Example 1, 566 (14%) of the patients were identified as exhibiting at least one of the noted 14 pairs of genetic variants. Using the biomarker analysis techniques described in Example 1, 2687 of the patients (65%) were determined to be CCP IgG positive, while 1451 of the patients were determined to be CCP IgG negative. Of the CCP IgG negative patients, 247 (17%) exhibited at least one of the 14 genetic variants.

This means, within the 4138 rheumatoid arthritis patient group, 57% of the patients are only CCP IgG positive, 8% of the patients are CCP IgG positive + genetic variant pair positive, 6% of the patients are only genetic variant pair positive, and 29% of the patients are only CCP IgG negative. Therefore, only using CCP IgG as a means for determining rheumatoid arthritis would have identified 65% of the patients, but by adding the genetic variant, 71% of the patients were identified. If patients having at least one pair of the genetic variants are diagnosed as at risk, prior to showing the CCP IgG biomarker, early treatment can be considered to prevent serious injury prior to the appearance of CCP IgG biomarker. Additionally, for patients having at least one pair of the genetic variants (i.e., a positive genetic score), closer monitoring of additional symptoms can be considered to prevent a delay in diagnosis of rheumatoid arthritis in CCP IgG-negative RA patients.

In conjunction with the rheumatoid arthritis patient group, whole blood and serum samples from 1328 healthy control subjects and 434 disease control subjects (systemic lupus erythematosus (SLE), Sjogren's syndrome, scleroderma and cancer patients) were also analyzed. The specificity of the genetic variant pair test was 95% against healthy control subjects (65 of the healthy control subjects showed positive results for the genetic variant pair rules) and 93% for the disease controls (31 of the disease controls showed positive results for the genetic variant pair rules).

While the present invention has been illustrated by the description of embodiments thereof and the above Example, and while the embodiments have been described in considerable detail, it is not the intention of the Applicant to restrict or in any way limit the scope of the appended claims to such detail. Additional advantages and modifications will readily appear to those skilled in the art. Therefore, the application, in its broader aspects, is not limited to the specific details and illustrative examples shown and described. Accordingly, departures may be made from such details without departing from the spirit or scope of the general inventive concept.

## Claims

1. A method for indicating a presence or absence of rheumatoid arthritis in an individual said method comprising determining the presence or absence of one or more of the following pairs of genetic variants in a biological sample obtained from the individual:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270,
wherein the presence of at least one of the pairs of the genetic variants in the biological sample is indicative of the presence of rheumatoid arthritis in said individual as compared to the absence of all of the pairs of genetic variants in the biological sample.

2. The method of claim 1, further comprising determining a concentration in a serum and/or plasma sample obtained from the individual of one or more of the biomarkers: anti-cyclic citrullinated peptide immunoglobulin G (CCP IgG), rheumatoid factor immunoglobulin M (RF IgM), anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and/or anti-rheumatoid arthritis 33 immunoglobulin M (RA33 IgM), wherein the presence of at least one of the biomarkers in a concentration level above its respective upper limit of normal in the serum and/or plasma sample is indicative of the presence of rheumatoid arthritis in said individual.

3. The method of claim 2, comprising determining the respective concentrations in a serum and/or plasma sample obtained from the individual of the biomarkers: anti-cyclic citrullinated peptide immunoglobulin G (CCP IgG) and rheumatoid factor immunoglobulin M (RF IgM).

4. The method of claim 2, comprising determining the respective concentrations in a serum and/or plasma sample obtained from the individual of one or more, such as all, of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM).

5. The method of claim 2, comprising determining the respective concentrations in a serum and/or plasma sample obtained from the individual of the biomarkers: anti-cyclic citrullinated peptide immunoglobulin G (CCP IgG), anti-cyclic citrullinated peptide immunoglobulin A (CCP IgA), rheumatoid factor immunoglobulin M (RF IgM), and rheumatoid factor immunoglobulin A (RF IgA).

6. The method of claim 5, comprising determining the respective concentrations in a serum and/or plasma sample obtained from the individual of the biomarkers: anti-rheumatoid arthritis 33 immunoglobulin A (RA33 IgA), anti-rheumatoid arthritis 33 immunoglobulin G (RA33 IgG), and/or anti-rheumatoid arthritis 33 immunoglobulin M (RA33 IgM).

7. The method of any one of claims 1-6, further comprising obtaining information regarding symptoms of arthralgia in said individual.

8. A computer-implemented method of determining a level of risk for developing rheumatoid arthritis in an individual, which method is executed by at least one processor in a computer, the method comprising the steps of:
determining the presence or absence in a biological sample obtained from the individual of one or more of the following pairs of genetic variants:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270; and
correlating the presence of at least one of the pairs of genetic variants in the biological sample with an increased risk of the individual developing rheumatoid arthritis as compared with a risk of the individual developing rheumatoid arthritis in the absence of all of the pairs of genetic variants in the biological sample.

9. The method of claim 8, further comprising
determining the respective concentrations in a serum and/or plasma sample obtained from the individual of the biomarkers: anti-cyclic citrullinated peptide immunoglobulin G (CCP IgG) and rheumatoid factor immunoglobulin M (RF IgM);
obtaining information regarding symptoms of arthralgia in said individual; and
correlating at least two of (a) the presence of at least one of the pairs of genetic variants in the biological sample, (b) the presence of at least one of the biomarkers in a concentration level above its respective upper limit of normal in the serum and/or plasma sample, and (c) the presence of symptoms of arthralgia in said individual, with an increased risk of the individual developing rheumatoid arthritis as compared with the risk of the individual developing rheumatoid arthritis in the absence of at least two of (a)-(c).

10. The method of claim 9, in the absence of (a) and (c), correlating (i) the presence of both of the biomarkers in concentration levels above their respective upper limits of normal, and/or (ii) the presence of CCP IgG in a concentration level more than three times its upper limit of normal, and/or (iii) the presence of RF IgM in a concentration level more than ten times its upper limit of normal with an increased risk of the individual developing rheumatoid arthritis as compared with the risk of the individual developing rheumatoid arthritis in the absence of (i), (ii) and (iii).

11. The method of claim 9, in the absence of (a) and (c), further comprising determining the respective concentrations of one or more of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM) in a serum and/or plasma sample obtained from the individual; and
correlating the presence of two or more of the biomarkers CCP IgG, RF IgM, CCP IgA, RF IgA, RA33 IgA, RA33 IgG and RA33 IgM in the serum and/or plasma sample in concentration levels above their respective upper limits of normal with an increased risk of the individual developing rheumatoid arthritis as compared with the risk of the individual developing rheumatoid arthritis in the absence of at least two of the biomarkers in concentration levels above their respective upper limits of normal in the serum and/or plasma sample.

12. The method of claim 9, in the absence of (a) and (c), further comprising determining the respective concentrations of one or more of the biomarkers: anti-cyclic citrullinated peptide immunoglobulin A (CCP IgA), rheumatoid factor immunoglobulin A (RF IgA), anti-rheumatoid arthritis 33 immunoglobulin A (RA33 IgA), anti-rheumatoid arthritis 33 immunoglobulin G (RA33 IgG), and anti-rheumatoid arthritis 33 immunoglobulin M (RA33 IgM) in a serum and/or plasma sample obtained from the individual; and
correlating (i) the presence of at least one biomarker selected from CCP IgG, CCP IgA, RF IgA, RA33 IgA, RA33 IgG and RA33 IgM in a concentration level more than three times its respective upper limit of normal, and/or (ii) the presence of RF IgM in a concentration level more than ten times its upper limit of normal with an increased risk of the individual developing rheumatoid arthritis as compared with the risk of the individual developing rheumatoid arthritis in the of absence of (i) and (ii).

13. The method of any one of claims 8-12, further comprising obtaining information regarding one or more of the following factors: the presence of obesity in said individual, the gender of the individual being female and the individual's regular smoking of tobacco, wherein the presence of one or more of the factors correlates to an increased risk of the individual developing rheumatoid arthritis as compared to a risk of the individual in developing rheumatoid arthritis in the absence of all of the factors.

14. The method of claim 1 or claim 8, wherein the individual has previously tested negative for the presence of the biomarker anti-cyclic citrullinated peptide immunoglobulin G (CCP IgG) in a serum and/or plasma sample in concentration level above its upper limit of normal, the individual has previously tested negative for the presence of the biomarker rheumatoid factor immunoglobulin M (RF IgM) in a serum and/or plasma sample in a concentration level above its upper limit of normal and/or the individual is a first degree relative of a person previously diagnosed with rheumatoid arthritis

15. The method of any one of the preceding claims, wherein the presence of at least one of the pairs of genetic variants in the biological sample is determined using polymerase chain reactions.

16. The method of any one of claims 2-7 and 9-15, wherein the concentration of one or more of the biomarkers is determined via immunoassay or, more specifically, fluorescence enzyme immunoassay and/or wherein the concentration of one or more of the biomarkers is determined with a microarray.

17. An assay panel of oligonucleotide primer pairs for multiplex polymerase chain reaction (PCR) allowing amplification and identification of the following genes in a biological sample:
rs1050152, rs3821236, rs1800629, rs25882, rs17367504, rs3027898, rs11574914, rs7403531, rs11889341, rs874881, rs2240340, rs4586, rs2014300, rs4700060, rs2847297, rs4810485, rs10181656, rs17375901, rs1045642, rs2298383 and rs387907270, optionally wherein said oligonucleotide primer pairs are provided on a genotyping array.

18. An assay system comprising the assay panel of claim 17 and means for sequencing amplicons from a multiplex PCR conducted using the panel.

19. An assay system of claim 18, further comprising a computer program product loadable into an internal memory of a computer, the computer program product comprising instructions which, when the computer program product is executed by a computer, cause the computer to correlate the presence of one or more of the following pairs of genetic variants in the sequenced amplicons to a risk of developing rheumatoid arthritis:
rs1050152 and rs3821236;
rs1800629 and rs25882;
rs17367504 and rs3027898;
rs11574914 and rs7403531;
rs11889341 and rs874881;
rs2240340 and rs7403531;
rs1800629 and rs4586;
rs2014300 and rs7403531;
rs1050152 and rs4700060;
rs2847297 and rs4810485;
rs10181656 and rs17375901;
rs17375901 and rs3821236;
rs1045642 and rs4700060; and
rs2298383 and rs387907270.

20. A computer program product loadable into an internal memory of a computer, the computer program product comprising instructions which, when the program is executed by the computer, cause the computer to carry out at least one of the correlating steps of the method of any one of claims 8-13.

21. Use of an assay panel of claim 17 or an assay system of claim 18, in an *in vitro* method of diagnosis, prognosis or assessment of rheumatoid arthritis in an individual.
